Europäisches Patentamt

European Patent Office

Office européen des brevets

(19)

(11) Veröffentlichungsnummer: **0 216 875**
**B1**

(12) **EUROPÄISCHE PATENTSCHRIFT**

(45) Veröffentlichungstag der Patentschrift: **30.05.90**

(21) Anmeldenummer: **86902272.3**

(22) Anmeldetag: **09.04.86**

(86) Internationale Anmeldenummer:
**PCT/AT86/00032**

(87) Internationale Veröffentlichungsnummer:
**WO 86/05996 23.10.86 Gazette 86/23**

(51) Int. Cl.⁵: **B 01 D 3/00,** C 12 F 1/00,
C 12 P 7/06

(54) VORRICHTUNG ZUR KONTINUIERLICHEN GEWINNUNG VON ETHANOL AUS VERZUCKERTEN AUSGANGSSTOFFEN.

(30) Priorität: **09.04.85 AT 1070/85**

(43) Veröffentlichungstag der Anmeldung:
**08.04.87 Patentblatt 87/15**

(45) Bekanntmachung des Hinweises auf die
Patenterteilung:
**30.05.90 Patentblatt 90/22**

(84) Benannte Vertragsstaaten:
**BE CH DE FR GB IT LI LU NL SE**

(56) Entgegenhaltungen:
**EP-A-0 114 161
DE-A-3 113 223
FR-A-2 482 979
FR-A-2 553 098**

**Rev.Gale de l'Electricitè, Vol. 89, number 3,
March 1982 see pages 221-222; figures 2A, 2B, 3**

(73) Patentinhaber: **VOEST-ALPINE
Aktiengesellschaft
Muldenstrasse 5
A-4020 Linz (AT)**

(72) Erfinder: **FALTEJSEK, Karl
Lüfteneggerstrasse 6
A-4020 Linz (AT)**
Erfinder: **CVITAS, Vilim
Voltastrasse 29
A-4040 Linz (AT)**
Erfinder: **HANKE, Reinhart
Annaberggasse 2
A-8700 Leoben (AT)**

(74) Vertreter: **Haffner, Thomas M., Dr. et al
Patentanwaltskanzlei Dipl.-Ing. Adolf
Kretschmer Dr. Thomas M. Haffner
Schottengasse 3a
A-1014 Wien (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Anordnung zur kontinuierlichen Gewinnung von Ethanol aus stärkehaltigen Ausgangsstoffen mit einer Einrichtung zum enzymatischen Verzuckern, einem nachgeschalteten Mischer zum Beladen von Gärhefe mit dem verzuckerten Substrat und einer Gärvorrichtung, aus welcher Ethanol über die Gasphase unter vermindertem Druck abziehbar ist. In der EP—A—114 161 wurde bereits vorgeschlagen, vergärbare Zuckerlösungen durch enzymatische Verzuckerung von stärkehältigen Ausgangsprodukten zu erzeugen. Neben einem thermischen Aufschluß in einem Autoklaven bei etwa 120°C wurde hiezu die enzymatische Verzuckerung bei Temperaturen von etwa 70°C und einem Wassergehalt von 70 bis 80 % vorgenommen. Anschließend an diese Verzuckerung erfolgte ein Eindicken mittels einer Zentrifuge bzw. eines Filters wobei der Zuckerlösung in der Folge Hefe zugesetzt wurde um Hefe mit dem vergärbaren Substrat zu beladen. Nach einer, beispielsweise durch Flotation, vorgenommene Abtrennung der beladenen Hefe wurde in der Folge einer Gärung durchgeführt, wobei unter Anwendung von Unterdruck der entstehende Alkohol destillativ abgetrennt wurde.

Die FR—A—2 482 979 beschreibt ein Destillationsverfahren für Alkohol, bei welchem der Energieaufwand für die Destillation durch Einsatz einer Wärmepumpe verringert werden soll.

Aus der nicht-vorveröffentlichten EP—A—155 578 ist ein Rektifikationsverfahren zu entnehmen, bei welchem der untere Teil der Kolonne beheizt und der obere Teil der Kolonne gekühlt wird. Die Heizung und Kühlung dieser Rektifikationskolonne sind durch eine Wärmepumpe miteinander gekoppelt.

Ausgehend von diesem Stand der Technik, zielt die vorliegende Erfindung nun darauf ab, den Energieaufwand zu verringern und die Abtrennung der einzelnen Fraktionen weiter zu verbessern, sowie das für die einzelnen Verfahrensstufen geforderte Temperaturniveau präziser einzuhalten. Zur Lösung dieser Aufgabe besteht die Erfindung im wesentlichen darin, daß ein Kältekreislauf vorgesehen ist, welcher einen Kompressor und wenigstens zwei Wärmetauscher enthält, von welchen einer als Verdampfer ausgebildet ist, daß dieser Kühlkreislauf Temperaturen zwischen kleiner als +5°, vorzugsweise -10°, und höchstens +60° aufweist und mit einem von Wasser oder Luft verschiedenen Kältekreislaufmedium betrieben ist, daß ein in Kältekreislaufrichtung im Anschluß an den Kompressor nachfolgender Wärmetauscher als Aufkocher für das Sumpfprodukt der Destillation geschaltet ist und der Verdampfer zur Kühlung des Destillates geschaltet ist und daß das aus der Destilliervorrichtung rückgewonnene Wasser, vorzugsweise unter Zwischenschaltung eines Wärmetauschers, zwischen Verzuckerung und Beladung mit Hefe, einem mit Dampf beaufschlagten Wärmetauscher zuführbar ist und der Verzuckerung rückführbar ist. Durch einen derartigen vom Wasserkühlkreislauf getrennten, gesonderten Kältekreislauf, welcher mit einem vom Wasser oder Luft verschiedenen Kühlmedium betrieben ist, läßt sich unter Verwendung eines Kompressors ein Temperaturniveau von Temperaturen unter 0°C bis höchstens +60°C vorgeben, welches mit der konventionellen Wasserkühlung nicht erreicht wird. Dieser zusätzliche Kältekreislauf enthält neben einem als Verdampfer ausgebildeten Wärmetauscher zur Kondensation des Destillates und einem Kompressor nun erfindungsgemäß wenigstens einen weiteren Wärmetauscher, welcher als Aufkocher für die zur Abtrennung des Alkohols verwendete Destilliereinrichtung eingesetzt wird. Durch den Verdampfer kann der Verlust an mit der Gasphase abziehendem Ethanol wesentlich herabgesetzt werden, da ein wesentlich geringeres Temperaturniveau vorgegeben werden kann.

Dadurch, daß das aus der Destilliervorrichtung rückgewonnene Wasser, vorzugsweise unter Zwischenschaltung eines Wärmetauschers zwischen Verzuckerung und Beladung mit Hefe einem mit Dampf beaufschlagten Wärmetauscher zugeführt, und der Verzuckerung rückgeführt wird, kann der Reinwasserbedarf der erfindungsgemäßen Einrichtung wesentlich verringert werden.

Der Kältekreislauf kann bei Verwendung von halogenierten Kohlenwasserstoffen, Alkoholen od.dgl. als Kältemedium in an sich bekannter Weise als geschlossener Kreislauf ausgebildet sein. Je nach Art des verwendeten Kältemediums kann es wünschenswert sein, das Kältemedium nach der Kompression auf eine Temperatur von unter 40°C abzukühlen. Eine derartige Abkühlung kann aber auch nach dem als Aufkocher für das Sumpfprodukt geschalteten Kondensator der Wärmeanlage vorgesehen sein. In vorteilhafter Weise ist vor dem Verdampfer ein weiterer Wärmetauscher vorgesehen, in welchem Kühlwasser das Kältekreislaufmedium auf Temperaturen von unter +40°C, vorzugsweise 20 bis 40°C, abkühlt.

Die Verzuckerung erfordert relativ hohe Temperaturen und zu diesem Zweck ist der mit Dampf beaufschlagte Wärmetauscher vorgesehen. In vorteilhafter Weise ist der Dampf für den mit Dampf beaufschlagten Wärmetauscher zumindest teilweise aus einem Abgaswärmetauscher des Antriebsaggregates, insbesondere einer Verbrennungskraftmaschine, für den Kompressor entnommen.

Im Zuge der biologischen Umsetzung entsteht $CO_2$ und dieses biologisch reine $CO_2$ kann im Rahmen der erfindungsgemäßen Vorrichtung in einfacher Weise rückgewonnen werden. Hiezu ist vorzugsweise an die Abzugsleitung der Destilliervorrichtung nach dem als Verdampfer ausgebildeten Wärmetauscher des Kältekreislaufes ein $CO_2$-Gasverdichter angeschlossen. In vorteilhafter Weise können einem derartigen $CO_2$-Gasverdichter auch die Gase aus der Saugleitung aus der Gärvorrichtung zugeführt werden, wofür in bevorzugter Weise gleichfalls ein als Verdampfer ausgebildeter Wärmetauscher des Kältekreislaufes zwischengeschaltet ist.

Neben der Möglichkeit als Kältemedium durch Kompression verflüssigbare Gase in geschlossenem Kreislauf einzusetzen, ergibt sich im Rahmen der erfindungsgemäßen Vorrichtung mit besonderem Vorteil die Möglichkeit als Kältekreislaufmedium eine Teilmenge des aus Destillation abgezogenen Alkohols einzusetzen, welcher zumindest teilweise zur Kühlung der aus der Destillation abgezogenen Gasphase in die Abzugsleitung aus der Destilliervorrichtung, vorzugsweise in eine Mischkammer, entspannt wird. Bei dieser Ausbildung des Kältekreislaufes handelt es sich genaugenommen um einen offenen Kreislauf, da das in den Kältekreislauf eingespeiste Kältemedium laufend der Destillation entnommen wird, und nach Entspannung mit dem gebildeten Alkohol abgezogen wird.

Zur weiteren Erniedrigung des Temperaturniveaus in der Abzugsleitung kann mit Vorteil der Verdampfer in der Abzugsleitung der Destilliervorrichtung einem Wasserkühler für das Destillat nachgeschaltet sein.

Zur Konstanthaltung der Temperatur des Mantels der Gärvorrichtung kann warmes Kühlwasser (Rücklauf) herangezogen werden, wofür ein gesonderter Wärmetauscher vorgesehen ist. In den Kältekreislauf ist vor oder nach dem Aufkocher für das Sumpfprodukt der Destillierkolonne ein mit Kühlwasser betriebener Unterkühler eingeschaltet, mit welchem das noch heiße komprimierte Kältemittel abgekühlt wird, bevor es dem Verdampfer zugeleitet wird. In einem derartigen Unterkühler wird Kühlwasser auf Temperaturen zwischen 30 und 45°C aufgewärmt, und ein auf diese Weise relativ zu Frischwasser wärmeres Wasser kann mit Vorteil zur Konstanthaltung der Temperatur des Mantels der Gärvorrichtung eingesetzt werden.

Die Erfindung wird nachfolgend anhand eines in der Zeichnung schematisch dargestellten Ausführungsbeispieles näher erläutert. In dieser zeigen Fig.1 eine erste Ausbildung mit einem geschlossenen Kältekreislauf, Fig. 2 eine abgewandelte Ausbildung mit einem gleichfalls geschlossenen Kältekreislauf und Fig. 3 einen Kältekreislauf unter Verwendung des aus der Destillation abgezogenen Alkoholes als Kältemittel.

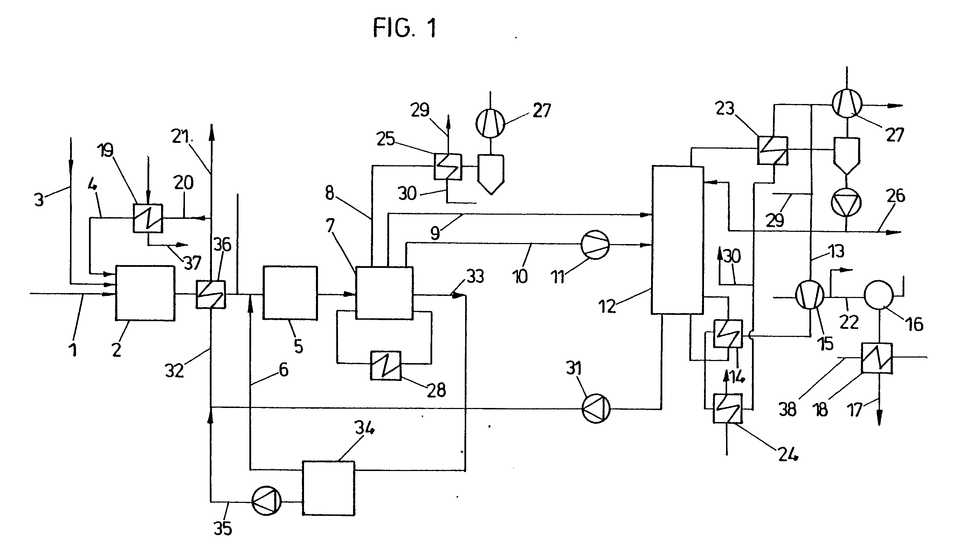

In Fig. 1 ist schematisch angedeutet, daß über eine Fördereinrichtung 1 Stärkemehl einer Verzuckerung 2 zugeführt wird. Dieser Verzuckerung wird weiters über eine Leitung 3 Enzymlösung, insbesondere auf etwa 95°C vorgewärmte Enzymlösung, zugesetzt. Zur Erzielung einer Temperatur im Bereich zwischen 70 und 90°C, welche durch Vorwärmung des Stärkemehles aus etwa 70°C mit Sicherheit erreicht werden kann, wird zusätzlich Wasser unter überatmosphärischen Druck bei 105°C über die Leitung 4 eingespeist. Bei Entspannung verdampft derartiges überhitztes Wasser, wodurch eine intensive und gleichmäßige Durchwärmung bei der Verzuckerung sichergestellt ist.

Das verzuckerte Produkt wird einer Flotation 5 unter Anwendung eines elektrischen Feldes unterworfen, wobei hier gleichzeitig über eine Fördereinrichtung 6 Hefe zugesetzt wird. Die beladene Hefe gelangt in eine mehrstufige Gärvorrichtung 7, aus welcher über eine Leitung 8 und ein Sauggebläse $CO_2$ und Wasserdampf abgezogen werden kann. Über die weiteren Gasableitungen 9 und 10 wird bereits ein mehr oder minder großer Anteil Ethanol abgefördert, wobei aus der letzten Gärstufe unter Anwendung eines gegenüber den vorangehenden Gärstufen weiter abgesenkten Druckes gearbeitet wird, wofür ein Sauggebläse 11 vorgesehen ist. Die endgültige Abtrennung des Alkohols erfolgt in einer Destillierkolonne 12.

Mit 13 ist ein gesonderter mit einem von Wasser und Luft verschiedenen Kältemedium betriebener Kühlkreislauf bezeichnet, welcher einen Wärmetauscher 14 für die Aufwärmung des Sumpfes der Destillierkolonne 12 und einen Kompressor 15 enthält. Als Kältemedium können hiebei fluorierte Kohlenwasserstoffe od.dgl. verwendet werden. Der Antrieb des Kompressors wird von einem Dieselmotor 16 geliefert, in dessen Abgasleitung 17 ein Wärmetauscher 18 für die Dampferzeugung eingeschaltet ist. Der Dampf aus diesem Wärmetauscher 18 wird einem Wärmetauscher 19 in der Wasserrückleitung 20 zugeführt, über welchen eine Teilmenge des rückgeführten Wassers geleitet wird. Eine andere Teilmenge des rückgeführten Wassers gelangt über eine Leitung 21 zur Enzymaufbereitung bzw. -lösung und in der Folge über die Leitung 3 in die Verzuckerung 2.

Die den Kompressor mit dem Motor verbindende Welle ist mit 22 bezeichnet.

In dem bei der Ausbildung nach Fig. 1 geschlossenen Kältekreislauf 13 sind neben dem Wärmetauscher 14 weitere Wärmetauscher 23, 24 und 25 eingeschaltet. Der Wärmetauscher 23 ist als Verdampfer und damit als Kopfkondensator für das aus der Destillierkolonne 12 abgezogene Gas ausgebildet, wobei überaus reines Ethanol abgeschieden und über eine Leitung 26 abgezogen werden kann. Bei dieser Gelegenheit abgezogenes $CO_2$ kann einem gesonderten Gasverdichter 27 zugeführt werden, wobei ein derartiger Gasverdichter 27 auch in die Gasableitung 8 aus der ersten Gärstufe der Gärvorrichtung 7 eingeschaltet ist.

Ein weiterer Wärmetauscher 24 dient zur Abkühlung des Kältemediums nach der Kompression auf Temperaturen von unter +40°C. Derartiges Kühlwasser kann weiters für die Kühlung des Kompressors 15 die Kühlung der Gasverdichter 27 oder in einem Wärmetauscher 28 verwendet werden. Der Wärmetauscher 28 stellt hiebei die Konstanthaltung der Temperatur des Mantels der Gärvorrichtung 7 sicher.

Die Anschlüsse für den als Verdampfer ausgebildeten Wärmetauscher 25 an den Kältekreislauf 13 sind im Kältekreislauf 13 mit 29 und 30 bezeichnet. Die entsprechenden Leitungen wurden aus Gründen der Übersichtlichkeit nicht in die Zeichnung eingetragen.

Aus der Destillierkolonne 12 kann Wasser auf einem Temperaturniveau von etwa 45°C abgezogen werden und über eine Pumpe 31 in die Wasserrückführungsleitung 32 geführt werden.

Aus der Gärvorrichtung 7 wird über eine Austragsvorrichtung 33 Hefe einer Heferegenerierung 34 zugeführt, wobei die regenerierte Hefe in der Folge wiederum der Flotation 5 aufgegeben werden kann. Hiebei abgetrenntes Wasser kann über die Leitung 35 der Wassersammelleitung 32 wieder zur Verfügung gestellt werden.

In die Wasserrückführungs- bzw. -sammelleitung 32 ist in der Folge noch ein Wärmetauscher 36 eingeschaltet, welcher in die Leitung zwischen der Verzuckerung und der Flotation eingeschaltet ist. Durch diesen Wärmetauscher wird das rückgeführte wasser auf etwa 95°C wiederum vorgewärmt und kann in der Folge der Enzymaufbereitung bzw. nach einer nochmaligen Aufwärmung der Verzuckerung rückgeführt werden.

Die aus dem mit Dampf beaufschlagten Wärmetauscher 19 über eine Kondenswasserleitung 37 abgezogene Wassermenge kann über den Anschluß 38 dem Abgaswärmetauscher 18 zugeführt werden, worauf in der Folge nur eine geringfügige neuerliche Erwärmung für die neuerliche Verwendung als Dampf im Wärmetauscher 19 erforderlich ist. Nach dem Inbetriebsetzen der Anlage kann somit die erforderliche Dampfmenge zur Gänze durch den Abgaswärmetauscher 18 zur Verfügung gestellt werden.

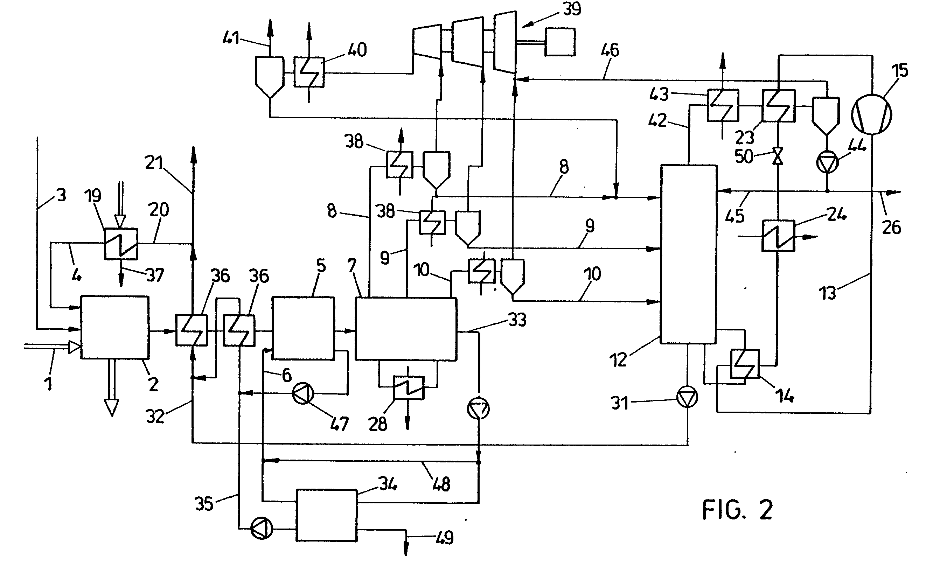

Bei der Ausbildung nach Fig. 2 sind in die Gasableitungen 8, 9 und 10 der Gärvorrichtung 7 Wasserkühler 38 eingeschaltet, worauf Ethanol und Wasser der Destilliervorrichtung 12 zugeführt werden. Die Gasabzugsleitungen werden einem mehrstufigem Gasverdichter 39 zugeführt. Nach neuerlicher Kühlung in einem mit Kaltwasser gespeisten Kühler 40 kann biologisch reines $CO_2$ über eine Leitung 41 abgezogen werden. In die Destilatabzugsleitung 42 ist ein erster mit Kühlwasser betriebener Kopfkondensator 43 eingeschaltet, bevor das abgekühlte Destillat dem als Verdampfer ausgebildeten Wärmetauscher 23 des Kältemittelkreislaufes 13 zugeführt wird. Die kondensierte flüssige phase enthält weitgehend reines Ethanol, welches bei Temperaturen von ca.5°C über die Leitung 26 abgezogen wird. In diese Leitung ist eine Pumpe 44 eingeschaltet, und es kann ein Teil des auf 5°C abgekühlten Ethanols über eine Leitung 45 in den Kopf der Destillationskolon- ne rückgeführt werden, um die Kondensation zu begünstigen. Die Gasphase gelangt über eine Leitung 46 an den mehrstufigen Gasverdichter 39, worauf auch aus diesem Gasanteil biologisch reines $CO_2$ abgeschieden wird.

Aus der Flotationsstufe 5 wird die abgereicherte Zuckerlösung über eine Pumpe 47 abgezogen und über einen zusätzlichen Wärmetauscher 36 gemeinsam mit dem Abwasser aus der Heferegenerierung der Enzymaufbereitung rückgeführt. Das durch den ersten Wärmetauscher 36 bereits vorgewärmte, teilweise zuckerhaltige Wasser gelangt hiezu in die Wasserrückführungs-bzw. Sammelleitung 32.

Ein Teil der aus der Gärvorrichtung 7 abgezogenen Hefe kann unter Umgehung der Heferegenerierung über eine By-Pass-Leitung 48 neuerlich in die Flotation rückgeführt werden. Die Überschußhefe aus der Heferegenerierung 34 wird über eine Leitung 49 abgezogen und kann einer Futtermittelproduktion zugeführt werden.

Im geschlossenen Kältemittelkreislauf ist vor dem Verdampfer üblicherweise eine Drossel eingeschaltet, welche in Fig. 2 mit 50 bezeichnet ist.

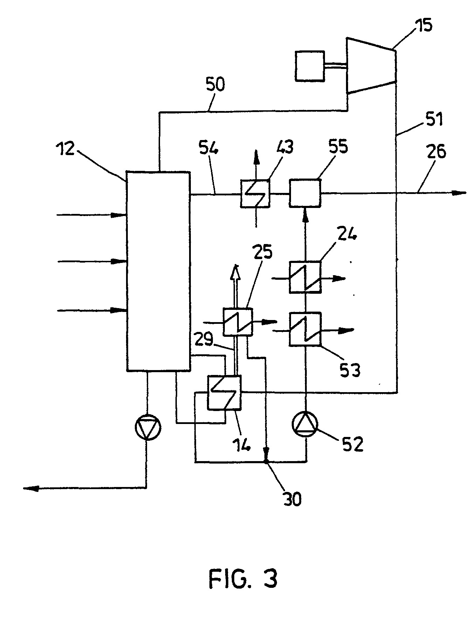

Bei der Ausbildung nach Fig. 3 ist ein offener Kältemittelkreislauf dargestellt. Aus der Destilliervorrichtung 12 wird über eine Kopfleitung 50 Alkohol abgezogen. Der Alkohol wird im Kompressor 15 komprimiert und verflüssigt und gelangt über die Kältemittelleitung 51 zum Aufkocher 14 für das Sumpfprodukt der Destilliervorrichtung 12. Zur Absenkung der Temperatur ist wiederum ein mit Kühlwasser betriebener Unterkühler 24 vorgesehen. Es ist weiters schematisch wiederum ein Wärmetauscher 25, welcher als Verdampfer ausgebildet ist, angedeutet. Nach dem Aufkocher 14 gelangt das Kältemedium über eine Pumpe 52 in einen Wärmetauscher 53, welcher als Ersatz für den Wärmetauscher 28 in den Fig. 1 und 2 zur Aufrechterhaltung einer Temperatur von etwa 40° bis 45° und damit zu einer Beheizung der Gärvorrichtung 7 dient.

Aus dem Kopf der Destilliervorrichtung wird über eine Leitung 54 Destillat abgezogen und in einem ersten Kopfkondensator 43, welcher wiederum mit Kühlwasser betrieben ist, vorgekühlt. Anschließend ist eine Mischkammer 55 vorgesehen, in welche das Kältemedium nach dem Unterkühler 24 entspannt wird, wodurch eine weitere starke Abkühlung des Destillates und der Kondensation des Alkohols erfolgt. Der Alkohol wird wiederum über die Leitung 26 abgezogen, wobei über diese Leitung 26 nunmehr unmittelbar aus der Destilliervorrichtung 12 abgezogener Alkohol gemeinsam mit Alkohol nach Durchlaufen des Kältemittelkreislaufes abgefördert wird. Der Kältemittelkreislauf wird kontinuierlich durch frisches über die Leitung 50 abgezogenes Destillat unter Zwischenschaltung des Kompressors 15 aufgefüllt.

**Patentansprüche**

1. Vorrichtung zur kontinuierlichen Gewinnung von Ethanol aus stärkehaltigen Ausgangsstoffen mit einer Einrichtung zum enzymatischen Verzuckern (2), einem nachgeschalteten Mischer zum Beladen von Gärhefe mit dem verzuckerten Substrat und einer Gärvorrichtung (7), aus welcher Ethanol über die Gasphase unter vermindertem Druck abziehbar ist, dadurch gekennzeichnet, daß ein Kältekreislauf (13) vorgesehen ist, welcher einen Kompressor (15) und wenigstens zwei Wärmetauscher (14, 23) enthält, von welchen einer (23) als Verdampfer ausgebildet ist, daß dieser Kühlkreislauf (13) Temperaturen zwischen kleiner als +5°, vorzugsweise -10°, und höchstens +60°C aufweist und mit einem von Wasser oder Luft verschiedenen Kältekreislaufmedium betrieben ist, daß ein in Kältekreislaufrichtung im Anschluß an den Kompressor (15) nachfolgender Wärmetauscher (14) als Aufkocher für das Sumpfprodukt einer Destillation (12) geschaltet ist und der Verdampfer (23) zur Kühlung des Destillates geschaltet ist, und daß das aus der Destilliervorrichtung (12) rückgewonnene Wasser, vorzugsweise unter Zwischenschaltung eines Wärmetauschers (36), zwischen Verzuckerung (2) und Beladung (5) mit Hefe, einem mit Dampf beaufschlagten Wärmetauscher (19) zuführbar ist und der Verzuckerung (2) rückführbar ist.

2. Vorrichtung nach Anspruch 1, dadurch gekennzeichnet, daß vor dem Verdampfer (23) ein weiterer Wärmetauscher (24) vorgesehen ist, in welchem Kühlwasser das Kältekreislaufmedium auf Temperaturen von unter +40°C, vorzugsweise 20 bis 40°C, abkühlt.

3. Vorrichtung nach Anspruch 1 oder 2, dadurch gekennzeichnet, daß der Dampf für den mit Dampf beaufschlagten Wärmetauscher (19) zumindest teilweise aus einem Abgaswärmetauscher (18) des Antriebsaggregates (16), insbesondere einer Verbrennungskraftmaschine, für den Kompressor (15) entnommen ist.

4. Vorrichtung nach einem der Ansprüche 1 bis 3, dadurch gekennzeichnet, daß an die Abzugsleitung der Destilliervorrichtung (12) nach dem als Verdampfer ausgebildeten Wärmetauscher (23) des Kältekreislaufes (13) ein $CO_2$-Gasverdichter (27) angeschlossen ist, an welchen eine, vorzugsweise gleichfalls einem als Verdampfer ausgebildeten Wärmetauscher (25) des Kältekreislaufes (13) nachgeschaltete, Saugleitung (8) aus einer Gärvorrichtung (7) mündet.

5. Vorrichtung nach einem der Ansprüche 1 bis 4, dadurch gekennzeichnet, daß als Kältekreislaufmedium eine Teilmenge des aus der Destillation (12) abgezogenen Alkohols eingesetzt wird, welcher zumindest teilweise zur Kühlung der aus der Destillation abgezogenen Gasphase in die Abzugsleitung aus der Destilliervorrichtung (12), vorzugsweise in eine Mischkammer (55), entspannt wird.

6. Vorrichtung nach einem der Ansprüche 1 bis 5, dadurch gekennzeichnet, daß der Verdampfer (23) in der Abzugsleitung (42) der Destilliervorrichtung (12) einem Wasserkühler (43) für das Destillat nachgeschaltet ist.

**Revendications**

1. Dispositif d'extraction continue d'éthanol de de produits féculents de base renfermant de l'amidon, comprenant un dispositif de saccharification enzymatique (2), un mélangeur, en aval, destiné au chargement de levure de fermentation avec le substrat saccharifié, ainsi qu'un dispositif de fermentation (7) duquel il est possible de soutirer, en phase gazeuse, de l'éthanol, sous une pression réduite, caractérisé en ce qu'il est prévu un cycle frigorifique (13) qui comprend un compresseur (15) et au moins deux échangeurs de chaleur (14,23), dont l'un (23) est conçu sous la forme d'un évaporateur, en ce que ce cycle frigorifique (13) présente des températures entre moins de +5°, de préférence -10°, et au plus +60°C et qui fonctionne avec un agent frigorigène différent de l'eau et de l'air, en ce qu'un échangeur de chaleur (14), faisant office de réchauffeur pour le résidu d'une unité de distillation (12), est prévu après le compresseur (15) dans le sens de fonctionnement du cycle frigorifique, ainsi qu'un évaporateur (23) pour le refroidissement du distillat, et en ce que l'eau extraite du dispositif de distillation (12) peut être amenée, de préférence avec interposition d'un échangeur de chaleur (36) entre la saccharification (2) et le chargement en levure (5), à un échangeur de chaleur (19) fonctionnant à la vapeur, et ramenée dans l'unité de saccharification (2).

2. Dispositif selon la revendication 1, caractérisée en ce que en amont de l'évaporateur (23), est prévu un autre échangeur de chaleur (24) dans lequel de l'eau de refroidissement abaisse la température de l'agent frigorigène à des températures inférieures à +40°C, de préférence 20 à 40°C.

3. Dispositif selon la revendication 1 ou 2, caractérisée en ce que la vapeur pour l'échangeur de chaleur (19) fonctionnant à la vapeur, est soutirée d'un échangeur de chaleur (18) sur les gaz d'échappement du dispositif d'entrainement (16), notamment un moteur à combustion, pour le compresseur (15).

4. Dispositif selon l'une des revendications 1 à 3, caractérisée en ce que à la conduite de soutirage de l'unité de distillation (12), après l'échangeur de chaleur (23) réalisé sous la forme d'un évaporateur, du cycle frigorifique (13), est raccordé un compresseur à gaz $CO_2$ (27), auquel arrive la conduite d'aspiration (8) d'un dispositif de fermentation (7), conduite de préférence raccordée en aval à un échangeur de chaleur (25) réalisé de même à la manière d'un évaporateur, du cycle frigorifique (13).

5. Dispositif selon l'une des revendications 1 à 4, caractérisée en ce que l'on utilise en guise d'agent frigorigène, une partie de l'alcool soutiré de l'unité de distillation (12) et détendu dans la conduite de soutirage de l'unité de distillation (12), de préférence dans une chambre de mélange (55), pour refroidir, au moins partiellement, la phase gazeuse soutirée de la distillation.

6. Dispositif selon l'une des revendications 1 à 5, caractérisée en ce que l'évaporateur (23) dans la conduite de soutirage (42) de l'unité de distillation (12) succède à un réfrigérant à eau (43) pour le distillat.

**Claims**

1. An apparatus for the continuous extraction of ethanol from raw materials containing starch, having a device for enzymatic saccharification (2), a mixer disposed downstream thereof for loading fermentation yeast with the saccharified substrate, and a fermentation vessel (7) from which ethanol can be drawn off under reduced pressure via the gaseous phase, characterised in that a cooling circuit (13) is provided which includes a compressor (15) and at least two heat exchangers (14, 23), one of which (23) is in the form of an evaporator, in that this cooling circuit (13) has temperatures of between less than +5°, preferably -10°, and at maximum +60°C and is operated with a cooling circuit medium other than water or air, in that a heat exchanger (14) is connected downstream of and following the compressor (15), in the direction of the cooling circuit, as a reboiler for the bottoms product of distillation (12) and the evaporator (23) is provided for cooling the distillate, and in that the water recovered from the distillation device (12), preferably with the interposition of a heat exchanger (36), can be fed to a heat exchanger (19) to which steam is admitted, between the saccharification (2) and loading (5) with yeast, and can be fed back to the saccharification (2).

2. An apparatus according to Claim 1, characterised in that upstream of the evaporator (23) there is provided a further heat exchanger (24), in which cooling water cools the cooling circuit medium to temperatures of below +40°C, preferably 20 to 40°C.

3. An apparatus according to Claim 1 or 2, characterised in that the steam for the heat exchanger (19), to which steam is admitted, is taken at least partly from an exhaust gas heat exchanger (18) of a drive unit (16), in particular an internal-combustion engine, for the compressor (15).

4. An apparatus according to any one of Claims 1 to 3, characterised in that a $CO_2$ gas compressor (27) is connected to the outlet duct of the distillation device (12) downstream of the heat exchanger (23) of the cooling circuit (13), which heat exchanger (23) is in the form of an evaporator, into which $CO_2$ gas compressor (27) an intake duct (8) from a fermentation device (7) discharges, which duct (8) is connected downstream of a heat exchanger (25) of said cooling circuit (13), which heat exchanger (25) is preferably also in the form of an evaporator.

5. An apparatus according to any one of Claims 1 to 4, characterised in that a portion of the alcohol drawn off from distillation (12) is used as the cooling circuit medium and is expanded at least partly for cooling the gaseous phase drawn off from the distillation into the outlet duct from the distillation device (12), preferably into a mixing chamber (55).

6. An apparatus according to any one of Claims 1 to 5, characterised in that the evaporator (23) in the outlet duct (42) of the distillation device (12) is connected downstream of a water cooler (43) for the distillate.

# FIG. 1

EP 0 216 875 B1

FIG. 2

EP 0 216 875 B1

EP 0 216 875 B1

FIG. 3

3